# EUROPÄISCHE PATENTSCHRIFT

(11) **EP 0 717 080 B1**
(45) Veröffentlichungstag und Bekanntmachung des Hinweises auf die Patenterteilung: **17.10.2001**
(21) Anmeldenummer: 95119108.9
(22) Anmeldetag: 05.12.1995
(51) Int. Cl.: C09B 11/20

(54) **Verfahren zur Herstellung von Triphenylmethanfarbmitteln**
Method for the preparation of triphenylmethan colorants
Procédé pour la préparation des colorants du type triphénylméthane

(30) Priorität: 14.12.1994 DE 4444472
(43) Veröffentlichungstag der Anmeldung: 19.06.1996
(73) Patentinhaber: Clariant GmbH, 65929 Frankfurt am Main (DE)
(72) Erfinder: Acs, Arpad, Dr., D-61440 Oberursel (DE); Remsperger, Hans-Josef, D-65439 Flörsheim (DE); Berger, Jürgen, D-65830 Kriftel (DE)

(56) Entgegenhaltungen:
- EP-A- 0 060 428
- FR-A- 1 196 870

## Beschreibung

Die vorliegende Erfindung liegt auf dem Gebiet der Triphenylmethanfarbmitteln.

Die Monosulfonsäuren von Triphenylmethanfarbmitteln haben große technische Bedeutung für die Herstellung blauer und insbesondere schwarzer Druckfarben, wofür sie sowohl als Pigmentpulver als auch in Form verschiedener Pigmentpräparationen, z.B. Flushpasten, eingesetzt werden.

Die Herstellung der in industriellem Maßstab produzierten Triphenylmethanfarbmittel erfolgt durch Monosulfierung der entsprechenden Farbbasen oder Farbbasensulfate in Schwefelsäure bestimmter Konzentration und Temperatur. Die zur Sulfonierung einsetzbaren Farbbasen erhält man durch Umsetzung von Rosanilinen mit primären aromatischen Aminen in Gegenwart saurer Katalysatoren, Vakuumdestillation der in der fertigen Schmelze enthaltenen flüchtigen Base bei Temperaturen um 150°C und Zerkleinerung der erkalteten Restschmelze (Fierz-David, Künstliche organische Farbstoffe, 1926, S. 262 und BIOS Final Report, 1433, S. 30, 31, 36) oder durch Überführung der nach den DE-A-1 098 652, DE-A-1 161 370 und DE-A-1 161 371 durch Umsetzung von 4,4',4"-Trichlortrityltetrachloraluminat mit Basen erhältlichen Farbbasenhydrochloride oder -tetrachloraluminate in die freien Farbbasen mittels Behandlung mit Alkohol und Alkalilauge (Fierz-David, Künstliche organische Farbstoffe, 1926, S. 264) oder mittels Behandlung mit Alkali in Gegenwart organischer Basen und anschließender Vakuumdestillation der Farbbasenlösung (BIOS Final Report, 1433, S. 32).

Die ebenfalls zur Monosulfierung einsetzbaren Farbbasensulfate erhält man durch Behandlung der in aromatischen Aminen oder in Gemischen aus aromatischen Aminen und nicht wasserlöslichen organischen Lösungsmitteln gelösten Farbbasen mit Schwefelsäure und anschließender Aufarbeitung des Farbbasensulfates aus der Fällungssuspension (DE-A-1 919 724).

Monosulfonsäuren von Triphenylmethanfarbstoffen sind auch ohne separaten Sulfierungsschritt erhältlich, wenn für die Herstellung der vorstehend genannten Farbbasentetrachloraluminate sulfogruppenhaltige aromatische Amine eingesetzt werden. Obwohl durch den Wegfall des Sulfierungsschrittes ein theoretisch kostengünstigeres und umweltfreundlicheres Herstellungsverfahren ermöglicht wird, haben die dabei aus aromatischen Sulfonsäure-Salzen (DE-A-1 098 652) oder aromatischen Sulfonsäuren (DE-A-3 108 720) gewonnenen Triphenylmethanpigmente jedoch bis heute keine technische und wirtschaftliche Bedeutung erlangt. So sind bisher nur Verfahren bekannt, die entweder bei der Synthese oder bei der Aufarbeitung des Reaktionsprodukts (DE-A-1 098 652, DE-A-3 108 720) des Einsatzes von wasserlöslichen organischen Lösemittein, beispielsweise Ethanol, Pyridin, Essigsäure, Aceton, Glykol, DMF oder DMSO, bedürfen, was bei einer industriellen Anwendung mit einer umständlichen, aufwendigen und kostspieligen Lösungsmittelregeneration verbunden wäre. Verzichtet man bei den genannten Reaktions- oder Aufarbeitungsstufen auf ein wasserlösliches organisches Lösemittel, so steigert sich die Viskosität des Reaktionsansatzes proportional zum Umsetzungsgrad mit den sulfogruppenhaltigen aromatischen Aminen bis hin zu einer kaum mehr handhabbaren klumpigen Konsistenz des gesamten Ansatzes. Tritt eine zunehmende Verfestigung schon während der Reaktion als Folge der Umsetzung des Trichlortrityltetrachloraluminats mit dem sulfogruppenhaltigen aromatischen Amin ein, so führt dies zu einer unvollständigen Umsetzung mit einem Anteil der Säuregruppe im Endprodukt, der merklich unter dem von durch Schwefelsäure sulfierten Triphenylmethanpigmenten liegt, oder zumindest zu einer inhomogenen Umsetzung. Dies äußert sich in einer nicht vollständigen Auflösbarkeit des Triphenylmethanpigments in verdünnter Alkalilauge, verbunden mit einer eingetrübten Farbe der Lösung und einem Rückstand bei der Filtration der alkalischen Lösung. Triphenylmethanpigmente mit solchen Lösungseigenschaften sind für die Präparierung zu qualitativ hochwertigen Druckfarben, wie sie die mit Schwefelsäure sulfierten Triphenylmethanbasen liefern, ungeeignet. Ein unzureichender Umsetzungsgrad führt zudem zu einer schlechten oder gar ganz ausbleibenden Wasserabspaltung bei der Herstellung von Flushpastenpräparationen und zu einem hohen Grobkornanteil, der in den Anwendungsdrucken eine Stippenbildung verursacht. Bei auf gleiche Farbstärke eingestellten Druckfarbenpasten besitzen die aus aromatischen Aminosulfonsäuren oder deren Salzen nach dem Stand der Technik erhaltenen Triphenylmethanpigmente drastisch höhere Viskositäten, die mit den üblichen anwendungstechnischen Mitteln kaum noch zu handhaben sind.

Auch bei der alkalischen Aufarbeitung der Aminschmelzen kommt es bei entsprechend hohem Umsatz zur Ausbildung einer sehr zähen bis hin zu einer fast steinartigen Konsistenz des Ansatzes, wenn nach den bisher bekannten Verfahren auf den Einsatz von zur Verflüssigung dienenden wasserlöslichen organischen Lösungsmitteln verzichtet wird. Die Triphenylmethanpigmente sind aus Suspensionen solcher Konsistenz nur äußerst schwer zu isolieren, da sich die Suspensionen mit herkömmlichen Aufarbeitungsmethoden nur sehr mühsam und innerhalb einer angemessenen Zeitdauer nicht vollständig homogenisieren lassen. Eine direkte saure Aufarbeitung der Aminschmelzen ist bislang nicht beschrieben.

Für die Herstellung von Triphenylmethanpigmenten ist daher ein Verfahren erforderlich, das auf allen Reaktions- und Aufarbeitungsstufen auf den Einsatz wasserlöslicher organischer Lösungsmittel verzichtet und gleichzeitig eine geeignete Rohpigmentqualität liefert, die sich beim Einsatz in Druckfarben durch hohe Farbstärke, geeignete Coloristik, gute rheologische Eigenschaften und einen niedrigen Grobkornanteil auszeichnet, Qualitätseigenschaften, wie sie bisher ausschließlich über die verfahrenstechnisch nachteilige Sulfierung der Triphenylmethan-Farbbasen mit Schwefelsäure erreichbar sind.

Es wurde gefunden, daß diese Aufgabe überraschenderweise gelöst werden kann, wenn bei der Synthese des Triphenylmethanfarbmittels die nachfolgend beschriebenen Verfahrensschritte durchgeführt werden.

Gegenstand der Erfindung ist ein Verfahren zur Herstellung von Triaminotriphenylmethanfarbmitteln, dadurch gekennzeichnet, daß man
a). eine Eisen-, Bor- oder Aluminiumtrihalogenidkomplexverbindung eines 4,4',4"-Trihalogentriphenylmethanhalogenids zunächst mit einem Salz einer aromatischen Aminosulfonsäure im molaren Verhältnis von 0,6:1 bis 1,4:1 bei einer Temperatur zwischen 130 und 180°C, bei einem Halogenwasserstoffpartialdruck von mindestens 1 bar, und in Gegenwart eines polaren, nicht wasserlöslichen organischen Lösemittels umsetzt, dann
b) die nach a) erhaltene Reaktionssuspension mit mindestens 5 Moläquivalenten eines aromatischen Amins der Formel (2) 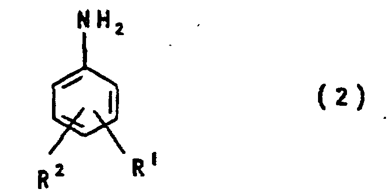 worin R¹ und R² gleich oder verschieden sind und die Bedeutungen Wasserstoff, Halogen, Methyl, Ethyl, Nitro, Methoxy oder Ethoxy haben, bei einer Temperatur zwischen 130 und 180°C umsetzt, dann
c) die nach b) erhaltene Aminschmelze, in Abwesenheit eines wasserlöslichen Lösemittels, entweder mit einer Mineralsäure auf einen pH-Wert zwischen 1 und 6 bringt, oder in Gegenwart eines viskositätssenkenden Additivs aus der Gruppe Alkalisalz eines Kondensationsproduktes aus Naphthalinsulfonsäure und Formaldehyd, Alkalisalz eines Copolymerisats aus Methacrylsäure und Maleinsäureanhydrid sowie ein Acetylendiol, mit einer Alkalilauge zunächst auf einen pH-Wert von mindestens 9 bringt, eine Phasentrennung durchführt, zur organischen Phase ein nicht wasserlösliches organisches Lösemittel zusetzt und anschließend durch Zugabe einer Mineralsäure einen pH-Wert zwischen 1 und 6 einstellt und schließlich,
d) das nach c) ausgefällte Farbmittel in üblicher Weise isoliert.

Das erfindungsgemäße Verfahren verläuft nach folgendem Reaktionsschema, das am Beispiel der Reaktionspartner Chlorbenzol, Aluminiumtrichlorid, 4-Chlorphenyltrichlormethan, Sulfanilsäure-Natriumsalz und m-Toluidin gezeigt ist:

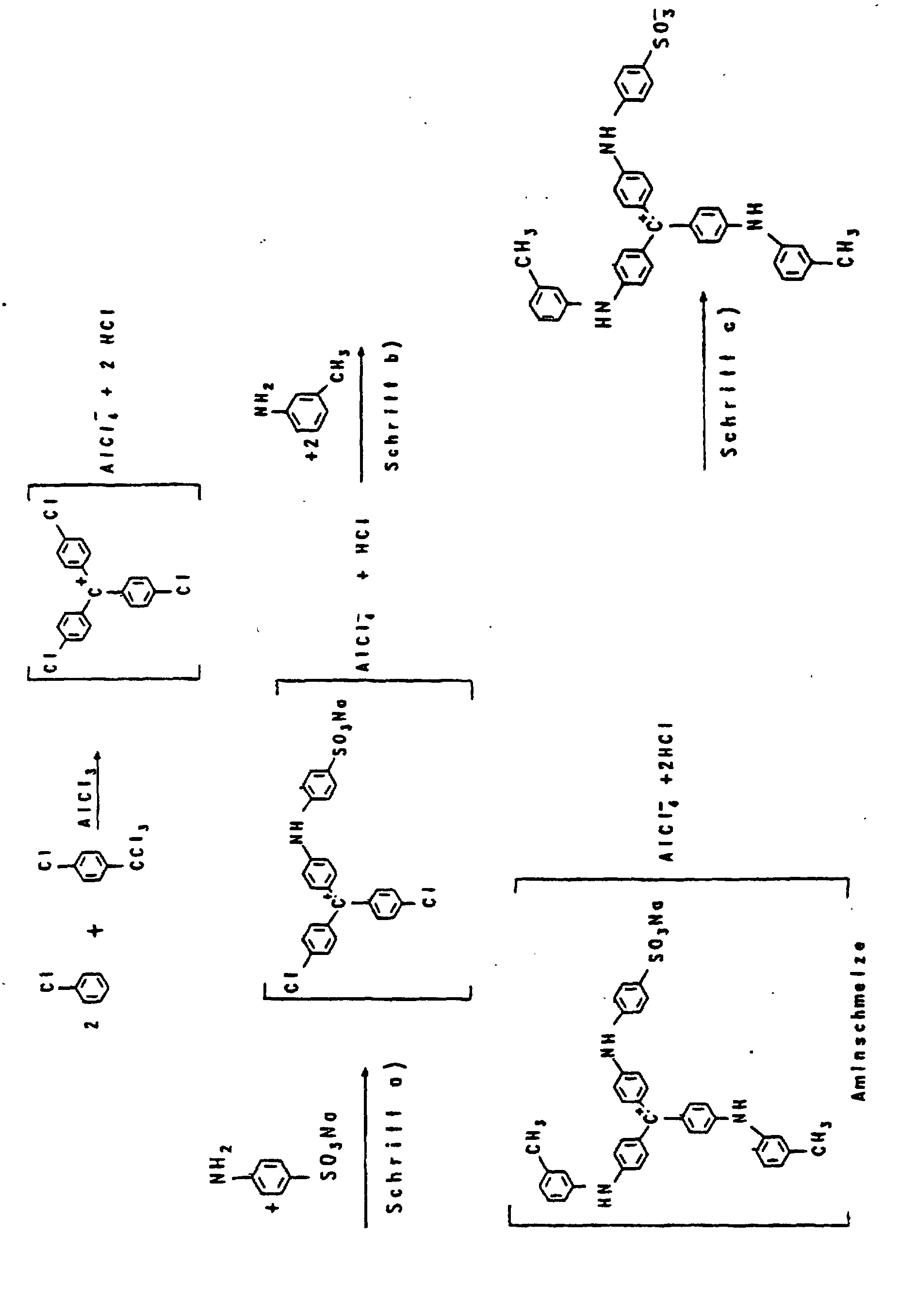

Die Herstellung der Eisen-, Bor- oder Aluminium-trihalogenidkomplexverbindung des 4,4',4"-Trihalogen-triphenylmethanhalogenids erfolgt nach üblichen, dem Fachmann allseits geläufigen Methoden durch Umsetzung eines Halogenbenzols mit einem p-Halogenbenzotrihalogenid und Eisen-, Bor- oder Aluminiumtrihalogenid. Als Halogen kommt in diesem Zusammenhang Chlor oder Brom, vorzugsweise Chlor, in Betracht.

Die so erhaltenen Komplexverbindungen werden mit 0,6 bis 1,4, vorzugsweise 1,0 bis 1,2, Molequivalenten eines Salzes einer aromatischen Aminosulfonsäure bei einer Temperatur zwischen 130 und 180°C, vorzugsweise 150 bis 170°C, in einem geschlossenen System zweckmäßigerweise bei dem sich einstellenden Überdruck und in Gegenwart eines mäßig polaren, nicht wasserlöslichen organischen Lösemittels, beispielsweise Chlorbenzol, Dichlorbenzol, Toluol, Xylole, Decalin oder Tetralin, zweckmäßigerweise während 1 bis 6 Stunden, bevorzugt etwa 3 Stunden, umgesetzt.

Als aromatische Aminosulfonsäuren sind beispielsweise Metanilsäure, Sulfanilsäure und ß-Naphthylamin-sulfonsäuren, wie 2-Naphthylamin-4-sulfonsäure, 2-Naphthylamin-5-sulfonsäure, 2-Naphthylamin-6-sulfonsäure, 2-Naphthylamin-7-sulfonsäure oder 2-Naphthylamin-8-sulfonsäure, von Interesse. Als Salze der genannten aromatischen Aminosulfonsäuren kommen Alkalisalze, Erdalkalisalze oder Ammoniumsalze in Betracht, bevorzugt Natriumsalze oder Kaliumsalze, insbesondere Natriumsalze. Besonders gute Ergebnisse werden erhalten, wenn die genannten Salze frei oder weitgehend frei von Kristallwasser sind.

Der sich im genannten Temperaturbereich einstellende Überdruck beträgt bei einer Füllhöhe des Gefäßes von 60 bis 80 % etwa 2 bis 4 bar. Der genannte Überdruck wird durch bei der Reaktion entstehendes Halogenwasserstoffgas mitverursacht und ist für die Durchführung des erfindungsgemäßen Verfahrens ausreichend. Es ist jedoch auch möglich, von außen zusätzlich Halogenwasserstoffgas aufzupressen. Eine Begrenzung der Höhe des Überdrucks ist nicht notwendig, mehr als 6 bar sind jedoch nicht sinnvoll. Es ist zweckmäßig, nach Zusammenführung der vorstehend genannten Reaktionspartner und Lösemittel in dem Reaktionsgefäß, z.B. einem Autoklaven, vor Beginn der Umsetzung bei einer Temperatur von 30 bis 60°C für eine Zeitdauer von 1 bis 30 Minuten ein Vakuum bis knapp über dem Siededampfdruck des eingesetzten Lösemittels anzulegen und anschließend die Umsetzung nach Verschluß des Autoklaven unter dem sich aufbauenden Druck bei den genannten Temperaturen durchzuführen. Es ist besonders vorteilhaft, wenn in axialer Richtung (axiale Rührverteilung) und bei einer Drehzahl zwischen 40 und 80, vorzugsweise 40 und 50, Umdrehungen pro Minute gerührt wird.

Das vorstehend beschriebene und als Schritt a) gekennzeichnete Verfahren zur Herstellung einer Verbindung der Formel (1) 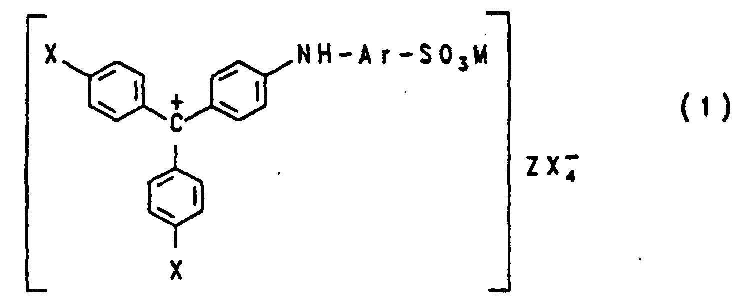 worin
- X: Halogen, vorzugsweise Chlor oder Brom, insbesondere Chlor,
- Z: Eisen, Bor oder Aluminium,
- M: ein Alkalimetall oder ein Erdalkalimetallequivalent oder eine Ammoniumgruppe, vorzugsweise Natrium oder Kalium, und
- Ar: einen Phenylen- oder Naphthylenrest bedeuten,
ist bislang noch nicht bekannt und ebenfalls Gegenstand der vorliegenden Erfindung. Die für Schritt a) beschriebenen bevorzugten Ausführungsformen gelten auch für die Herstellung der Verbindung der Formel (1).

Sollen Triaminotriphenylmethanfarbmittel hergestellt werden, so wird die Verbindung der Formel (1) in Form der aus Schritt a) erhaltenen Reaktionssuspension mit mindestens 5, vorzugsweise mit 5 bis 30, insbesondere mit 7 bis 15, Molequivalenten eines oder mehrerer aromatischer Amine der vorstehend genannten Formel (2) bei einer Temperatur zwischen 130 und 180°C, vorzugsweise 150 und 170°C, für eine Zeit von zweckmäßigerweise 1/2 bis 8 Stunden umgesetzt.
Bevorzugte Amine der Formel (2) sind Anilin, m-Toluidin, p-Toluidin, m-Anisidin, p-Anisidin, 2-, 3- und 4-Chloranilin, Nitroanilin, 2-, 3- und 4-Bromanilin. Während oder nach dieser Umsetzung kann das polare, nicht wasserlösliche organische Lösemittel teilweise oder vollständig entfernt werden, zweckmäßig durch Abdestillieren unter vermindertem Druck. Die Destillationsbedingungen sollten dabei so gewählt werden, daß das aromatische Amin überwiegend in der Aminschmelze verbleibt.

Das so erhaltene Reaktionsprodukt ist eine Schmelze, die im nächsten Schritt c) in Abwesenheit eines wasserlöslichen Lösemittels entweder mit einer vorzugsweise 5 bis 80 gew.-%igen, besonders bevorzugt 10 bis 20 gew.-%igen, Mineralsäure, beispielsweise Schwefelsäure, Salzsäure oder Phosphorsäure, insbesondere Schwefelsäure, oder mit einer vorzugsweise 10 bis 50 gew.-%igen, besonders bevorzugt 15 bis 30 gew.-%igen, Alkalilauge, beispielsweise Natronlauge oder Kalilauge, vorzugsweise Natronlauge, zersetzt wird.

Im Falle der sauren Zersetzung der Schmelze wird bei einer Temperatur zwischen 30 und 90°C, vorzugsweise 70 und 90°C, für eine Zeitdauer von zweckmäßigerweise 1 bis 3 Stunden umgesetzt. Die Menge der zugegebenen Mineralsäure wird dabei so bemessen, daß sich nach der Zersetzung der Aminschmelze ein pH-Wert zwischen 1 und 6 einstellt.

Im Falle der basischen Zersetzung der Aminschmelze wird bevorzugt bei einer Temperatur zwischen 50 und 100°C, vorzugsweise 80 und 100°C, für eine Zeitdauer von zweckmäßigerweise 1 bis 3 Stunden und in Gegenwart eines viskositätssenkenden Additivs umgesetzt. Die Menge der zugegebenen Alkalilauge wird dabei so bemessen, daß sich bei der Zersetzung der Aminschmelze ein pH-Wert von mindestens 9 einstellt. Besagtes Additiv ist ein Alkalisalz der Kondensationsprodukte aus Naphthalinsulfonsäure und Formaldehyd, der Copolymerisate aus Methacrylsäure und Maleinsäureanhydrid oder ein Acetylendiol und kann in Mengen von 0,1 bis 10 Gew.-%, vorzugsweise 0,5 bis 5 Gew.-%, bezogen auf das Gewicht des Triaminotriphenylmethanfarbmittels, eingesetzt werden. Das Rohpigment oder der Farbstoff wird anschließend nach Ansäuern der alkalischen Lösung, vorzugsweise mit Schwefelsäure, bei einem pH-Wert zwischen 1 und 6 direkt oder aus der abgetrennten und mit einem nicht wasserlöslichen organischen Lösemittel verdünnten organischen Phase ausgefällt. Das zum Verdünnen dienende nicht wasserlösliche organische Lösemittel wird zweckmäßigerweise in einer Menge von mindestens 200 Teilen pro 70 Teile Ausbeute an Farbmittel eingesetzt.

Die nach saurer oder alkalischer Zersetzung erhaltenen Fällungen des Triphenylmethanpigments werden durch Filtration und Waschung isoliert und im Anschluß an bekannte, beispielsweise in DE-A-41 14 863 beschriebene Finishmethoden zu geeigneten Pigmentpräparationen weiterverarbeitet.

Die erfindungsgemäß hergestellten Triphenylmethanfarbmittel erfüllen die für eine technisch durchführbare Verfahrensweise und die an die Qualität des Farbmittels gestellten Anforderungen. Sie zeigen nicht mehr die nach den bisherigen Verfahren auftretenden Nachteile im Verfahrensablauf und in der Qualität gegenüber den bisherigen Aminosulfonsäure- oder Aminosulfonsäuresalz-Verfahren. So führen die unter a) genannten Verfahrensbedingungen überraschenderweise zu einer erheblichen Verbesserung der Viskosität und damit der Homogenität und des Umsetzungsgrades der Reaktionspartner, was sonst nur mit einer drastischen Erhöhung der Lösemittelmenge, die ein Vielfaches der Ansatzgröße betrüge, erreichbar wäre. Die Endviskosität des Ansatzes bei einer Temperatur von 130°C liegt in einem Bereich von wasserflüssig bis honigartig bei einem Umsetzungsgrad zwischen 70 und 90 % und gleichzeitig homogener Sulfierverteilung. Erreicht wird ein Sulfiergrad von 0,7 bis 1,1. Ohne Einstellung der unter a) genannten Bedingungen liegt die Endviskosität bei der gleichen Temperatur zwischen hochpastös bis nahezu wachsartig, was zu einem Gehalt an Sulfogruppen von lediglich 0,4 bis 0,6, und in jedem Fall einer sehr uneinheitlichen "Sulfierverteilung" mit den bereits oben beschriebenen Nachteilen führt.

In den nachfolgenden Beispielen bedeuten "Teile" Gewichtsteile.

### Beispiel 1:

Zur Reaktionssuspension des aus 194 Teilen Chlorbenzol, 18 Teilen Aluminium(III)-chlorid und 30 Teilen p-Chlorbenzotrichlorid hergestellten 4,4',4"-Trichlortritylaluminiumtetrachlorids werden 28 Teile wasserfreies Sulfanilsäure-Natriumsalz hinzugefügt und in einem geschlossenen Autoklaven nach Anlegen eines 15-minütigen Vorvakuums von 0,4 bar 3 Stunden bei 160°C mittels eines in axialer Richtung verteilenden Rührers bei einer Drehzahl von 50 bis 60 Umdrehungen pro Minute gerührt. Nach Abkühlung auf unter 130°C, Entspannung des Autoklaven und Zugabe von 200 Teilen m-Toluidin wird der Reaktionsansatz unter vollständigem Abdestillieren des überschüssigen Chlorbenzols 3 Stunden bei 160°C und gegen Ende bei einem Vakuum von 0,4 bar geschmolzen. Die Schmelze wird anschließend mit 480 Teilen 10 gew.-%iger Schwefelsäure 1 Stunde bei 80°C zersetzt. Das ausgefallene, filtrierte und salzfrei gewaschene Rohpigment (Ausbeute 80 Teile) wird durch alkalische Umlösung feinverteilt und mit einem geeigneten Firnis, wie beispielsweise in DE-A-41 14 863 beschrieben, zu einer grünstichig blauen Druckfarbe verarbeitet. Die Druckfarbe eignet sich zum Schönen von Ruß und wegen ihres gegenüber einem mit Schwefelsäure sulfierten Triphenylmethanpigment reineren Farbtons auch zum Blaudruck.

### Beispiel 2

Eine Mischung aus einer 4,4',4"-Trichlortrityltetrachloraluminat-Suspension und wasserfreiem Metanilsäure-Na-Salz als Sulfosäurekomponente wird umgesetzt sowie die nachfolgende Schmelzreaktion mit p-Toluidin als aromatischem Amin wird in den Mengenverhältnissen und gemäß den Bedingungen nach Beispiel 1 durchgeführt. Die Schmelze wird anschließend in einer Mischung aus 33 Teilen Natriumhydroxid, 100 Teilen Wasser und 1 Teil eines Natriumsalzes eines Kondensationsproduktes aus Naphthalinsulfonsäure und Formaldehyd 3 Stunden lang bei 95°C zersetzt und das Rohpigment durch Versetzen der bei 85 bis 95°C abgetrennten organischen Phase mit 10 gew.-%iger Schwefelsäure auf einen pH-Wert zwischen 1 und 6 ausgefällt. Das mit einer Ausbeute von 80 Teilen angefallene Rohpigment liefert nach der in Beispiel 1 beschriebenen Präparierung eine Druckfarbe mit grünstichig blauem Farbton und oben beschriebenem Eignungsprofil.

### Beispiel 3

Es wird wie in Beispiel 2 verfahren, wobei als Additiv ein Natriumsalz eines Copolymerisats aus Methacrylsäure und Maleinsäureanhydrid oder als Additiv ein Acetylendiol eingesetzt wird. Man erhält ein Triphenylmethanpigment in der in Beispiel 2 beschriebenen Qualität.

### Beispiel 4

Pigmente mit ähnlichen Eigenschaften, wie unter den Beispielen 1 und 2 beschrieben, werden in analoger Weise durch Einsatz von p-Toluidin, 2-, 3- oder 4-Chlor-Anilin, Nitroanilin, m- oder p-Anisidin oder deren Gemischen hergestellt.

### Beispiel 5

Die unter Beispiel 1 erhaltene Reaktionsmischung aus 4,4',4"-Trichlortrityltetrachloraluminat und wasserfreiem Sulfanilsäure-Na-Salz wird mit 20 Teilen m-Toluidin 1 Stunde bei 130°C und anschließend mit weiteren 100 Teilen Anilin 2 Stunden bei 160°C unter gleichzeitigem Abdestillieren eines Chlorbenzol/ Anilin-Gemischs geschmolzen. Die Reaktionsschmelze wird zunächst mit weiteren 50 Teilen Anilin verdünnt und anschließend in 200 Teilen der in Beispiel 2 beschriebenen Wasser/Natriumhydroxid/Additiv-Mischung 2 Stunden bei 95°C alkalisch zersetzt. Danach werden weitere 200 Teile Wasser hinzugesetzt, die bei 85 bis 95°C abgetrennte organische Phase mit 300 Teilen Chlorbenzol verdünnt und durch langsame Zugabe von 90 Teilen 30 gew.-%iger Schwefelsäure das anilinsulfathaltige Rohpigment ausgefällt. Man suspendiert die abfiltrierte und mit Chlorbenzol gewaschene Fällung in 500 Teilen Wasser, treibt das überschüssige Chlorbenzol durch Wasserdampfdestillation aus und trennt das Anilinsulfat durch Filtration und Waschung ab. Ausbeute: 70 Teile. Mit einer nach Beispiel 1 durchgeführten Präparation erhält man eine rotstichige, besonders farbstarke blaue Druckfarbe, die sich besonders zum Schönen von schwarzer Druckfarbe eignet.

## Patentansprüche

1. Verfahren zur Herstellung einer Verbindung der Formel (1) 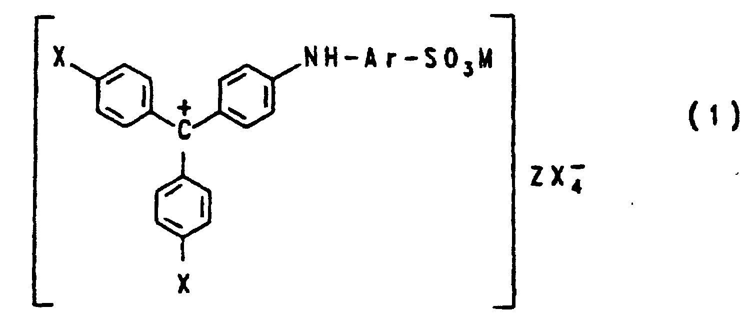 worin
X Halogen,
Z Eisen, Bor oder Aluminium,
M ein Alkalmetall oder ein Erdalkalimetallequivalent oder eine Ammoniumgruppe, und
Ar einen Phenylen- oder Naphthylenrest bedeuten,
**dadurch gekennzeichnet, daß** man eine Eisen-, Bor-, oder Aluminiumtrihalogenidkomplexverbindung eines 4,4',4"-Trihalogentriphenylmethanhalogenids zunächst mit einem Salz einer Aminophenylsulfonsäure oder einem Salz einer Aminonaphthalinsulfonsäure im molaren Verhältnis von 0.6:1 bis 1,4:1 bei einer Temperatur zwischen 130 und 180°C, bei einem Halogenwasserstoffpartialdruck von mindestens 1 bar, und in Gegenwart eines polaren, nicht wasserlöslichen organischen Lösemittels umsetzt.

2. Verfahren nach Anspruch 1, **dadurch gekennzeichnet, daß**
X Chlor oder Brom, vorzugsweise Chlor,
Z Eisen oder Aluminium und
M Natrium oder Kalium bedeuten.

3. Verfahren zur Herstellung von Triaminotriphenylmethanfarbmitteln,
**dadurch gekennzeichnet, daß** man
a) eine Eisen-, Bor- oder Aluminiumtrihalogenidkomplexverbindung eines 4,4',4"-Trihalogentriphenylmethanhalogenids zunächst mit einem Salz einer aromatischen Aminosulfonsäure im molaren Verhältnis von 0,6:1 bis 1,4:1 bei einer Temperatur zwischen 130 und 180°C, bei einem Halogenwasserstoffpartialdruck von mindestens 1 bar und in Gegenwart eines polaren, nicht wasserlöslichen organischen Lösemittels umsetzt, dann
b) die nach a) erhaltene Reaktionssuspension mit mindestens 5 Moläquivalenten eines aromatischen Amins der Formel (2) 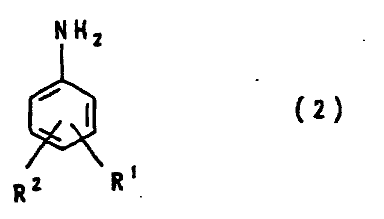 worin R¹ und R² gleich oder verschieden sind und die Bedeutungen Wasserstoff, Halogen, Methyl, Ethyl, Nitro, Methoxy oder Ethoxy haben, bei einer Temperatur zwischen 130 und 180°C umsetzt, dann
c) die nach b) erhaltene Aminschmelze in Abwesenheit eines wasserlöslichen Lösemittels, entweder durch Zugabe einer Mineralsäure auf einen pH-Wert zwischen 1 und 6 bringt, oder in Gegenwart eines viskositätssenkenden Additivs aus der Gruppe ein Alkalisalz eines Kondensationsproduktes aus Naphthalinsulfonsäure und Formaldehyd, ein Alkalisalz eines Copolymerisats aus Methacrylsäure und Maleinsäureanhydrid sowie ein Acetylendiol; mit einer Alkalilauge zunächst auf einen pH-Wert von mindestens 9 bringt, eine Phasentrennung durchführt, zur organischen Phase ein nicht wasserlösliches organisches Lösemittel zusetzt und anschließend durch Zugabe einer Mineralsäure einen pH-Wert zwischen 1 und 6 einstellt, und schließlich,
d) das nach c) ausgefällte Farbmittel in üblicher Weise isoliert.

4. Verfahren nach mindestens einem der Ansprüche 1 bis 3, **dadurch gekennzeichnet, daß** man eine Eisen- oder Aluminiumtrichlorkomplexverbindung eines 4,4',4"-Trichlortriphenylmethanchlorids einsetzt.

5. Verfahren nach mindestens einem der Ansprüche 1 bis 4, **dadurch gekennzeichnet, daß** man ein Alkalisalz, vorzugsweise das Natriumsalz, der Metanilsäure oder der Sulfanilsäure oder einer ß-Naphthylamin-sulfonsäure einsetzt.

6. Verfahren nach Anspruch 5, **dadurch gekennzeichnet, daß** das Alkalisalz der besagten Säuren kristallwasserfrei ist.

7. Verfahren nach mindestens einem der Ansprüche 1 bis 6, **dadurch gekennzeichnet, daß** man die Umsetzung mit dem Salz der aromatischen Aminosulfonsäure in einem geschlossenen System bei einer Temperatur zwischen 150 und 170°C und bei dem sich einstellenden Überdruck durchführt.

8. Verfahren nach mindestens einem der Ansprüche 1 bis 7, **dadurch gekennzeichnet, daß** man die Umsetzung mit dem Salz der aromatischen Aminosulfonsäure unter Rühren mit einer Drehzahl zwischen 40 und 80 Umdrehungen pro Minute und bevorzugt bei axialer Rührverteilung durchführt.

9. Verfahren nach mindestens einem der Ansprüche 3 bis 8, **dadurch gekennzeichnet, daß** das aromatische Amin der Formel (2) Anilin, m-Toluidin, p-Toluidin, m-Anisidin, p-Anisidin, 2-, 3- oder 4-Chloranilin, 2-, 3- oder 4-Bromanilin oder Nitroanilin ist.

10. Verfahren nach mindestens einem der Ansprüche 3 bis 9, **dadurch gekennzeichnet, daß** man im Verlauf oder nach Beendigung der Umsetzung b) das polare, nicht wasserlösliche organische Lösemittel teilweise oder vollständig entfernt.

11. Verfahren nach mindestens einem der Ansprüche 3 bis 10, **dadurch gekennzeichnet, daß** man den Verfahrensschritt c) mit 5 bis 80 gew.-%iger Schwefelsäure bei einer Temperatur zwischen 30 und 90°C durchführt.

12. Verfahren nach mindestens einem der Ansprüche 3 bis 10, **dadurch gekennzeichnet, daß** man den Verfahrensschritt c) mit 10 bis 50 gew.-%iger Natronlauge und in Gegenwart von 1 bis 10 Gew.-%, bezogen auf das Gewicht des Triaminotriphenylmethanfarbmittels, des viskositätssenkenden Additivs durchführt.

## Claims

1. A process for the preparation of a compound of the formula (1) 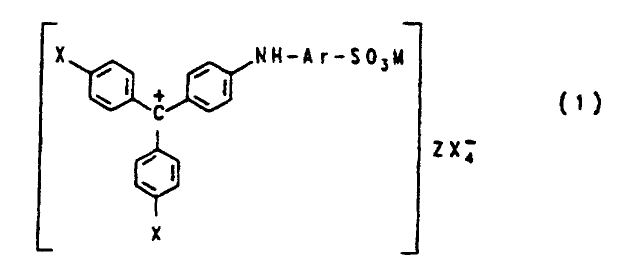 in which
X is halogen,
Z is iron, boron or aluminum,
M is an alkali metal or alkaline earth metal equivalent or an ammonium group and
Ar is a phenylene or naphthylene radical, which comprises first reacting an iron, boron or aluminum trihalide complex compound of a 4,4',4"-trihalotriphenylmethane halide with a salt of an aminophenylsulfonic acid or a salt of an aminonaphthalene sulfonic acid in a molar ratio of 0.6:1 to 1.4:1 at a temperature between 130 and 180°C, under a hydrogen halide partial pressure of at least 1 bar and in the presence of a polar, water-insoluble organic solvent.

2. The process as claimed in claim 1, wherein
X is chlorine or bromine, preferably chlorine,
Z is iron or aluminum and
M is sodium or potassium.

3. A process for the preparation of a triaminotriphenylmethane coloring agent, which comprises
a) first reacting an iron, boron or aluminum trihalide complex compound of a 4,4',4"-trihalotriphenylmethane halide with a salt of an aromatic aminosulfonic acid in a molar ratio of 0.6:1 to 1.4:1 at a temperature between 130 and 180°C, under a hydrogen halide partial pressure of at least 1 bar and in the presence of a polar, water-insoluble organic solvent, subsequently
b) reacting the reaction suspension obtained according to a) with at least 5 molar equivalents of an aromatic amine of the formula (2) 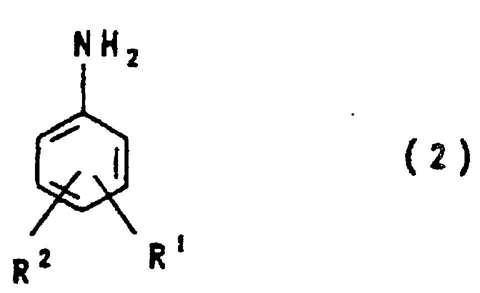 in which R¹ and R² are identical or different and are hydrogen, halogen, methyl, ethyl, nitro, methoxy or ethoxy, at a temperature between 130 and 180°C, subsequently
c) either bringing the amine melt obtained according to b), in the absence of a water-soluble solvent, to a pH of between 1 and 6 by addition of a mineral acid or first bringing it to a pH of at least 9 with an alkali metal hydroxide solution in the presence of a viscosity-lowering additive selected from the group consisting of an alkali metal salt of a condensation product from naphthalenesulfonic acid and formaldehyde, an alkali metal salt of a copolymer of methacrylic acid and maleic anhydride, and an acetylenediol carrying out a phase separation, adding a water-insoluble organic solvent to the organic phase and then bringing this to a pH of between 1 and 6 by addition of a mineral acid, and finally
d) isolating the coloring agent precipitated according to c) in the customary manner.

4. The process as claimed in at least one of claims 1 to 3, wherein an iron- or aluminum-trichloro complex compound of a 4,4',4"-trichlorotriphenylmethane chloride is employed.

5. The process as claimed in at least one of claims 1 to 4, wherein an alkali metal salt, preferably the sodium salt, of metanilic acid or of sulfanilic acid or of a β-naphthylamine-sulfonic acid is employed.

6. The process as claimed in claim 5, wherein the alkali metal salt of the said acids is free from water of crystallization.

7. The process as claimed in at least one of claims 1 to 6, wherein the reaction with the salt of the aromatic aminosulfonic acid is carried out in a closed system at a temperature between 150 and 170°C and at the autogenous increased pressure.

8. The process as claimed in at least one of claims 1 to 7, wherein the reaction with the salt of the aromatic aminosulfonic acid is carried out while stirring at a speed of between 40 and 80 revolutions per minute, preferably with axial stirring distribution.

9. The process as claimed in at least one of claims 3 to 8, wherein the aromatic amine of formula (2) is aniline, m-toluidine, p-toluidine, m-anisidine, p-anisidine, 2-, 3- or 4-chloroaniline, 2-, 3- or 4-bromoaniline or nitroaniline.

10. The process as claimed in at least one of claims 3 to 9, wherein the polar, water-insoluble organic solvent is partly or completely removed in the course of or after the end of reaction b).

11. The process as claimed in at least one of claims 3 to 10, wherein process step c) is carried out with 5 to 80% strength by weight sulfuric acid at a temperature between 30 and 90°C.

12. The process as claimed in at least one of claims 3 to 10, wherein process step c) is carried out with 10 to 50% strength by weight sodium hydroxide solution and in the presence of 1 to 10% by weight, based on the weight of the triaminotriphenylmethane coloring agent, of the viscosity-lowering additive.

## Revendications

1. Procédé pour la préparation d'un composé de formule (1) 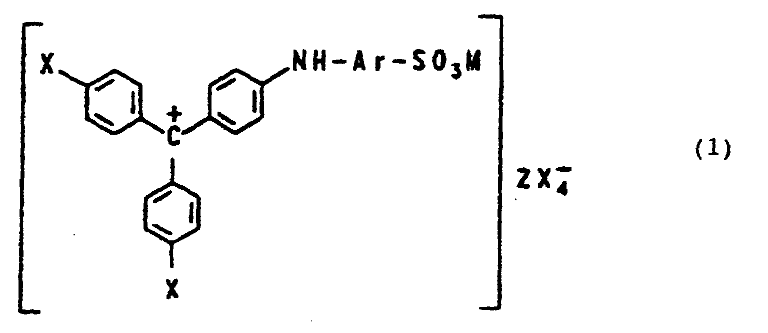 où
X représente un atome d'halogène,
Z représente un atome de fer, de bore ou d'aluminium,
M représente un métal alcalin ou un équivalent de métal alcalino-terreux ou un groupe ammonium, et
Ar représente un reste phénylène ou naphtylène,
**caractérisé en ce qu'**on fait réagir un composé complexe de trihalogénure de fer, de bore ou d'aluminium d'un halogénure de 4,4',4"-trihalogénotriphénylméthane d'abord avec un sel d'un acide aminophénylsulfonique ou un sel d'un acide aminonaphtalènesulfonique dans un rapport molaire de 0,6:1 à 1,4:1, à une température comprise entre 130 et 180°C, à une pression partielle d'un halogénure d'hydrogène d'au moins 1 bar, et en présence d'un solvant organique non hydrosoluble, polaire.

2. Procédé selon la revendication 1, **caractérisé en ce que**
X représente un atome de chlore ou de brome, de préférence de chlore,
Z représente un atome de fer ou d'aluminium et
M représente un atome de sodium ou de potassium.

3. Procédé pour la préparation de colorants de triaminotriphénylméthane, **caractérisé en ce que**
a) on fait réagir un composé complexe d'un trihalogénure de fer, de bore ou d'aluminium d'un halogénure de 4,4',4"-trihalogénotriphénylméthane, d'abord sur un sel d'un acide aminosulfonique aromatique dans un rapport molaire de 0,6:1 à 1,4:1, à une température comprise entre 130 et 180°C, à une pression partielle d'halogénure d'hydrogène d'au moins 1 bar et en présence d'un solvant organique non hydrosoluble, polaire, puis,
b) on fait réagir la suspension réactionnelle obtenue selon a) avec au moins 5 équivalents molaires d'une amine aromatique de formule (2) 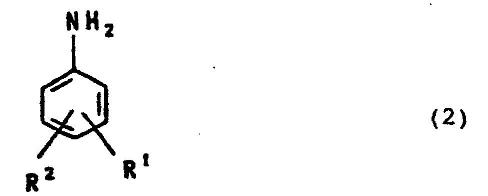 où R¹ et R² sont identiques ou différents et représentent un atome d'hydrogène, un atome d'halogène, des groupes méthyle, éthyle, nitro, méthoxy ou éthoxy, à une température comprise entre 130 et 180°C, puis
c) dans la matière fondue d'amine obtenue selon b), en l'absence d'un solvant hydrosoluble, soit on porte le pH par ajout d'un acide minéral à une valeur comprise entre 1 et 6, soit, en présence d'un additif réduisant la viscosité pris dans le groupe d'un sel alcalin d'un produit de condensation de l'acide naphtalènesulfonique et du formaldéhyde, d'un sel alcalin d'un copolymère de l'acide méthacrylique et de l'anhydride maléique ainsi que d'un acétylènediol, on porte d'abord la valeur de pH à au moins 9 au moyen d'une lessive alcaline, on effectue une séparation de phases, on ajoute à la phase organique un solvant organique non hydrosoluble et ensuite, par ajout d'un acide minéral, on établit une valeur de pH comprise entre 1 et 6, et finalement,
d) on isole de façon usuelle le colorant précipité selon c).

4. Procédé selon au moins l'une des revendications 1 à 3, **caractérisé en ce qu'**on utilise un composé complexe de trichlorure de fer ou d'aluminium d'un chlorure de 4,4',4"-trichlorotriphénylméthane.

5. Procédé selon au moins l'une des revendications 1 à 4, **caractérisé en ce qu'**on utilise un sel alcalin, de préférence le sel sodique, de l'acide métanilique ou de l'acide sulfanilique ou d'un acide β-naphtylaminosulfonique.

6. Procédé selon la revendication 5, **caractérisé en ce que** le sel alcalin dudit acide est exempt d'eau de cristallisation.

7. Procédé selon au moins l'une des revendications 1 à 6, **caractérisé en ce qu'**on met en oeuvre la réaction avec le sel de l'acide aminosulfonique aromatique dans un système fermé à une température comprise entre 150 et 170° et de la surpression qui s'établit.

8. Procédé selon au moins l'une des revendications 1 à 7, **caractérisé en ce qu'**on met en oeuvre la réaction avec le sel de l'acide aminosulfonique aromatique sous agitation avec un nombre de tours compris entre 40 et 80 tours par minute et de préférence sous une répartition d'agitation axiale.

9. Procédé selon au moins l'une des revendications 3 à 8, **caractérisé en ce que** l'amine aromatique de formule (2) est l'aniline, la m-toluidine, la p-toluidine, la m-anisidine, la p-anisidine la 2-, 3-ou 4-chloraniline, la 2-, 3- ou 4-bromaniline ou la nitroaniline.

10. Procédé selon au moins l'une des revendications 3 à 9, **caractérisé en ce qu'**on élimine partiellement ou complètement le solvant organique non hydrosoluble, polaire au cours ou après avoir terminé la réaction b).

11. Procédé selon au moins l'une des revendications 3 à 10, **caractérisé en ce qu'**on met en oeuvre le stade de procédé c) à l'aide d'acide sulfurique d'une concentration de 5 à 80 % en masse à une température comprise entre 30 et 90°C.

12. Procédé selon au moins l'une des revendications 3 à 10, **caractérisé en ce qu'**on met en oeuvre le stade de procédé c) à l'aide de lessive de soude ayant une concentration de 10 à 50 % en masse et en présence de 1 à 10 % en masse, par rapport au poids du colorant de triaminotriphénylméthane, de l'additif réduisant la viscosité.
